# EUROPÄISCHE PATENTANMELDUNG

(11) **EP 4 566 649 A1**
(43) Veröffentlichungstag der Anmeldung: **11.06.2025**
(21) Anmeldenummer: 24216951.4
(22) Anmeldetag: 02.12.2024
(51) Int. Cl.: A61M 16/08, A61M 16/01, A61M 16/20

(54) **VORRICHTUNG ZUR KEIMABWEHR IN EINEM BEATMUNGSSYSTEM**

(30) Priorität: 05.12.2023 DE 102023133947
(71) Anmelder: Löwenstein Medical Technology S.A., 2557 Luxembourg (LU)
(72) Erfinder: Schregel, Christian-Georg, 61169 Friedberg (DE)
(74) Vertreter: Löwenstein Medical IP

(57) **Zusammenfassung**

Die Erfindung betrifft eine Vorrichtung zur Keimabwehr in einem Beatmungssystem, wobei das Beatmungssystem neben der Vorrichtung ein Beatmungsgerät und ein Atemgasleitungssystem zum Leiten von Atemgas hin zum und/oder weg vom Beatmungsgerät umfasst, wobei die Vorrichtung eine Leitung zum Leiten des Atemgases zwischen dem Beatmungsgerät und dem Atemgasleitungssystem umfasst, wobei die Leitung einen ersten Leitungsabschnitt mit einem ersten Anschluss zum Anschließen der Leitung an das Beatmungsgerät, einen zweiten Leitungsabschnitt mit einem zweiten Anschluss um Anschließen der Leitung an das Atemgasleitungssystem und einen den ersten Leitungsabschnitt mit dem zweiten Leitungsabschnitt verbindenden dritten Leitungsabschnitt umfasst, wobei der dritte Leitungsabschnitt als Keimbarriere ausgebildet ist, um zu verhindern, dass Keime aus dem ersten Leitungsabschnitt in den zweiten Leitungsabschnitt und/oder aus dem zweiten Leitungsabschnitt in den ersten Leitungsabschnitt gelangen.

## Beschreibung

### Technisches Gebiet

Die Erfindung betrifft eine Vorrichtung zur Keimabwehr in einem Beatmungssystem. Des Weiteren betrifft die Erfindung ein Beatmungssystem mit einer solchen Vorrichtung. Das Beatmungssystem ist hierbei zur Narkotisierung und/oder zur Beatmung ausgebildet.

### Stand der Technik

Vorrichtungen zur Narkotisierung und/oder zur Beatmung, auch Anästhesie-Arbeitsplätze genannt, sind üblicherweise so aufgebaut, dass die Atemgase in einen Kreislauf geführt werden. Dafür werden verbrauchte Gase wie beispielsweise Sauerstoff (O₂) dem Kreislauf zugeführt und Kohlendioxid (CO₂) entfernt. In solch einem Kreislauf können volatile Anästhetika kontrolliert zugegeben und abgeschieden werden. Bedingt durch mindestens eine Atemgasquelle, beispielsweise in Form eines Gebläses, und Rückschlagventile fließt das Atemgas in einer definierten Richtung. Die Inspirationsluft wird über den inspiratorischen Zweig des Atemgaskreislaufs zum Patienten geführt. Nach der Inspiration gelangt die Exspirationsluft des Patienten über den exspiratorischen Zweig wieder in das Kreissystem und auch in das Gebläse.

Beim Betrieb von Anästhesie-Arbeitsplätzen kann durch mindestens ein Ventil, insbesondere durch ein Überdruckventil wie das sogenannte APL-Ventil (APL = *adjustable pressure limit; "*einstellbare Druckgrenze"), Gas aus dem Patientenkreislauf entsorgt werden, welches vom Patienten bereits ein- und wieder ausgeatmet wurde. Da dieses Gas mit volatilen Anästhetika angereichert ist, wird es in der Regel durch ein sogenanntes Anästhesiegasfortleitungssystem (kurz auch AGFS) abgeleitet und entsorgt.

Das Anästhesiegasfortleitungssystem besteht in der Regel aus festen Verrohrungen in der Krankenhauswand, in denen ein konstanter Unterdruck herrscht, um das Patientengas abzusaugen. Da die Exspirationsluft potenziell auch Pathogene aus der Lunge des Patienten enthalten kann, werden im Normalbetrieb HEPA-Filter verwendet, um diese zurückzuhalten. Diese halten allerdings nur 99,95% der Keime zurück.

Somit kann ein Teil der Keime über das APL-Ventil in die Rohre des Anästhesiegasfortleitungssystems gelangen. Durch Kondensation von Wasser aus der Exspirationsluft des Patienten kann in den Rohren eine potenzielle Basis für Verkeimung entstehen. Eine Aufbereitung der Rohre ist nicht einfach und im Krankenhausbetrieb eher unüblich. Daher besteht die Möglichkeit, dass sich im Anästhesiegasfortleitungssystem Keime ansammeln.

Da sich Bakterien auch entgegen einer gerichteten Luftströmung in Form eines Biofilms ausbreiten können, ist es möglich, dass Keime aus dem Anästhesiegasfortleitungssystem in Richtung des APL-Ventils aufsteigen und somit auch wieder in den Atemgaskreislauf und zum Patienten gelangen.

### Offenbarung der Erfindung

Eine Aufgabe der vorliegenden Erfindung kann darin gesehen werden, eine Vorrichtung bereitzustellen, die verhindert, dass Keime in den Atemkreislauf des Patienten gelangen. Eine weitere Aufgabe der Erfindung kann darin gesehen werden, ein entsprechend verbessertes Beatmungssystem bereitzustellen.

Diese Aufgaben werden durch die Gegenstände der unabhängigen Ansprüche gelöst nämlich mit einer Vorrichtung des Anspruchs 1 sowie mit einem Beatmungssystem gemäß Anspruch 15.

Weiterbildungen und vorteilhafte Ausführungsformen der Erfindung sind Gegenstand der Unteransprüche. Die Unteransprüche betreffen verschiedene voneinander unabhängige, vorteilhafte Weiterbildungen und Ausgestaltungen der vorliegenden Erfindung, deren Merkmale vom Fachmann im Rahmen des technisch Sinnvollen frei miteinander kombiniert werden können. Dies gilt insbesondere auch über die Grenzen der verschiedenen Anspruchskategorien hinaus. Die Beschreibung charakterisiert und spezifiziert die Erfindung insbesondere im Zusammenhang mit den Figuren zusätzlich.

Ein erster Aspekt der Erfindung betrifft eine Vorrichtung zur Keimabwehr in einem Beatmungssystem gemäß Anspruch 1. Das Beatmungssystem umfasst neben der Vorrichtung ein Beatmungsgerät und ein Atemgasleitungssystem zum Leiten von Atemgas hin zum und/oder weg vom Beatmungsgerät.

Die Vorrichtung umfasst eine Leitung zum Leiten des Atemgases zwischen dem Beatmungsgerät und dem Atemgasleitungssystem. Die Leitung umfasst einen ersten Leitungsabschnitt mit einem ersten Anschluss zum Anschließen der Leitung an das Beatmungsgerät, einen zweiten Leitungsabschnitt mit einem zweiten Anschluss zum Anschließen der Leitung an das Atemgasleitungssystem und einen den ersten Leitungsabschnitt mit dem zweiten Leitungsabschnitt verbindenden dritten Leitungsabschnitt. Der dritte Leitungsabschnitt ist als Keimbarriere ausgebildet, um zu verhindern, dass Keime aus dem ersten Leitungsabschnitt in den zweiten Leitungsabschnitt und/oder aus dem zweiten Leitungsabschnitt in den ersten Leitungsabschnitt gelangen.

Der Begriff "Keime" kann sämtliche Organismen umfassen, die Auswirkungen auf den Patienten haben können. Die Keime können sich in der Luft befinden und/oder in einem Biofilm eingebettet sein. Biofilme bestehen aus einer wässrigen Schicht, in der Populationen von Keimen lebens- und vermehrungsfähig existieren können.

Die Keime können beispielsweise mindestens einen der folgenden Keimtypen umfassen: Mikroorganismen, ein- oder mehrzellige Organismen, Kleinstlebewesen, Bakterien, Pilze, Algen, Parasiten, Prionen, Protisten, Viren, Viroide oder deren Derivate oder Vorstufen wie beispielsweise Pilzsporen, Sporen, Allergene, Toxine und dergleichen. Unter Keimen kann auch ein "Biofilm" verstanden werden. Die Keime können insbesondere Krankheitserreger oder Pathogene umfassen.

Unter "Keimabwehr" oder "Keimbarriere" kann verstanden werden, dass die Ausbreitung von Keimen abgewehrt wird, also unterbunden, oder zumindest gestört oder gehemmt und somit zeitlich verzögert wird. Die Keimabwehr kann sowohl die Abwehr einer Ausbreitung von Keimen über die Luft als auch die Abwehr einer Ausbreitung von Keimen in Form eines Biofilms umfassen.

Unter "Beatmungsgerät" kann eine Vorrichtung zur Narkotisierung und/oder zur Beatmung verstanden werden. Das Beatmungsgerät kann eine Beatmungsfunktion und/oder eine Anästhesiefunktion aufweisen. Das Beatmungsgerät kann somit als reines Beatmungsgerät und/oder als Anästhesiegerät verwendet werden.

Unter "Beatmung" kann eine (künstliche) Beatmung, eine Atemunterstützung, eine Atemtherapie, eine Atemdiagnose, eine Hustenunterstützung, eine Sauerstoffliefernde Therapie wie beispielweise eine High-Flow-Therapie, eine Inhalationsnarkose und eine Kombination aus mindestens zwei dieser Beispiele verstanden werden.

Somit kann ein Patient unter Verwendung des Beatmungsgerätes beatmet oder in der Atmung unterstützt werden und alternativ oder zusätzlich auch unter Narkose gehalten werden. Insbesondere kann das Beatmungsgerät auch mit Anästhesiegasen wie volatilen Anästhetika betrieben werden und somit einer Inhalationsanästhesie dienen.

Unter "Beatmungsgerät" können allgemein sämtliche Geräte verstanden werden, die einen Patienten oder sonstigen Anwender bei der natürlichen Atmung unterstützen und/oder die Beatmung übernehmen und/oder einer Atemtherapie dienen und/oder einer Inhalationsnarkose dienen und/oder anderweitig auf die Atmung des Patienten oder Anwenders einwirken. Patient und Anwender werden hierin synonym verwendet und bezeichnen jedwedes Individuum, das mit einem Beatmungsgerät beatmet und/oder narkotisiert wird.

Das Beatmungsgerät kann beispielsweise mindestens einen der folgenden Gerätetypen umfassen: ein Narkose- oder Anästhesiegerät (auch Anästhesie-Arbeitsplatz genannt), ein klinisches oder außerklinisches Beatmungsgerät, ein Notfall-Beatmungsgerät, ein sauerstofflieferndes Gerät, ein Atemtherapiegerät, ein CPAP-, APAP- oder BiLevel-Gerät, ein Highflow-Therapiegerät, ein Diagnosesystem, ein Hustentherapiegerät, eine Hustenmaschine oder eine Kombination aus mindestens zwei dieser Beispiele.

Zur Verbindung mit einem Patienten kann an das Beatmungsgerät ein Patienteninterface angeschlossen werden. Unter "Patienteninterface" kann allgemein ein Peripheriegerät zur Interaktion mit einem Lebewesen verstanden werden. Insbesondere kann das Patienteninterface zu Therapie- und/oder Diagnosezwecken in Verbindung mit dem Beatmungsgerät ausgebildet sein, beispielsweise als Atemmaske. Unter "Atemmaske" kann beispielsweise eine Nasenmaske, eine Nasenpolstermaske, eine Nasenbrille, eine Sauerstoffbrille, eine Full-Face-Maske, eine Totalface-Maske, eine Trachealtube oder -kanüle oder eine Kombination aus mindestens zwei dieser Beispiele verstanden werden.

Das Beatmungsgerät und das Patienteninterface können über eine Leitung miteinander verbunden sein. Diese Leitung kann als ein Schlauch oder Schlauchsystem ausgebildet sein. Das Schlauchsystem kann beispielsweise ein Zweischlauchsystem sein und mindestens einen Inspirationsschlauch in Kombination mit mindestens einem Exspirationsschlauch umfassen. In diesem Falle kann der Patient über den Inspirationsschlauch des Schlauchsystems und das Patienteninterface mit einem Atemgas versorgt werden und über den Exspirationsschlauch des Schlauchsystems ausatmen. Somit kann das Ausatemgas über den Exspirationsschlauch in das Beatmungssystem rückgeführt werden und es kann mindestens ein geschlossener Kreislauf bestehen. Ein geschlossener Kreislauf besteht, wenn das Ausatemgas - nach einer Aufbereitung - wieder für die Inspiration verwendet wird. Bei Verwendung eines Zweischlauchsystems kann ein Y-Stück verwendet werden, über das Inspirationsschlauch und Exspirationsschlauch mit dem Patienteninterface verbindbar sind.

Unter "Atemgas" kann ein einzuatmendes und/oder ausgeatmetes Gas oder Gasgemisch verstanden werden. Das Atemgas kann beispielsweise normale Atemluft aus der Umgebung, (reiner) Sauerstoff, ein Anästhesiegas(gemisch) oder mit Sauerstoff und/oder einem Anästhesiegas(gemisch) angereicherte Atemluft sein.

Unter "Leitung" kann beispielsweise ein (flexibler) Schlauch, ein (starres) Leitungsrohr oder eine Kombination aus beidem verstanden werden. Im Lumen der Leitung kann das Atemgas aufgenommen sein und geleitet werden. Die Leitung kann Anschlüsse zur (fluidischen) Verbindung mit weiteren Leitungen oder anderen Komponenten umfassen. Über die Anschlüsse kann eine luftdichte Verbindung ermöglicht sein.

Die Leitung umfasst den ersten Anschluss und den zweiten Anschluss. Der erste Anschluss kann mit einem Atemgasauslass des Beatmungsgeräts koppelbar sein. Alternativ oder zusätzlich kann der zweite Anschluss mit einem Atemgaseinlass des Atemgasleitungssystems koppelbar sein.

Der "Atemgasauslass" des Beatmungsgerätes kann beispielsweise ein Ventil, insbesondere ein Überdruckventil sein oder umfassen. Das Überdruckventil kann beispielsweise als APL-Ventil zur Regelung eines inspiratorischen Drucks ausgebildet sein. Das Atemgas kann über den Atemgasauslass aus einem Gaskreislauf des Beatmungsgerätes in die Vorrichtung und/oder das Atemgasleitungssystem abgeleitet werden, wenn der Druck den (voreingestellten) inspiratorischen Druck übersteigt.

Bei dem Atemgasleitungssystem kann es sich beispielsweise um ein sogenanntes Atemgasfortleitungssystem (auch AGFS genannt) handeln, mit dem das Atemgas aus dem Beatmungsgerät aufgenommen und kontrolliert abgeleitet oder aufbereitet werden kann. Über das Atemgasfortleitungssystem kann überschüssiges Atemgas aus einem zu einem Patienten führenden Gaskreislauf entnommen werden. Somit können potenziell umwelt- oder gesundheitsschädliche Stoffe kontrolliert entnommen, aufbereitet und/oder entsorgt werden. Ein solches Atemgasfortleitungssystem kann insbesondere dann zum Einsatz kommen, wenn das Atemgas volatile Anästhetika enthält.

Der als Keimbarriere ausgebildete dritte Leitungsabschnitt bietet den Vorteil, dass eine Ausbreitung von Keimen, z.B. in Form eines Biofilmes, mit geringem Aufwand kontrolliert verhindert oder zumindest vermindert oder zeitlich verzögert werden kann. Die Keimbarriere kann als ein Bauteil im Beatmungssystem angesehen werden, in dem die Ausbreitung von Keimen verlangsamt wird, da dort für Keime lebensfeindliche Bedingungen geschaffen sind, da weder Wasser noch Nährstoffe vorhanden sind.

Somit kann ein sicherer Betrieb des Beatmungssystems - im Vergleich zu einer Ausführungsform ohne eine solche Keimbarriere - über einen deutlich längeren Zeitraum gewährleistet werden. Dies verringert wiederum die Wartungs- und Instandhaltungskosten.

Die Keimbarriere kann ermöglichen, im Rahmen einer Risikoabschätzung klar zu definieren, wann im ungünstigsten anzunehmenden Fall (Worst-Case-Szenario) eine Verkeimung in das Beatmungsgerät und/oder in das Atemgasleitungssystem gelangen könnte. Zu diesem Zwecke kann in einem Testlauf mithilfe von Testkeimen eine Ausbreitungsgeschwindigkeit von Keimen in der Vorrichtung gemessen werden. Unter Hinzuziehung der Gesamtlänge der Keimbarriere kann eine zeitliche Aufbereitungsempfehlung ermittelt werden, die beispielsweise derart gewählt ist, dass die Aufbereitung erfolgt, bevor ein theoretisch vorhandener Biofilm zu 50% durch die Keimsperre hätte wachsen können. Die Keimbarriere kann somit derart ausgestaltet sein, dass dem Anwender eine zeitliche Aufbereitungsempfehlung ausgegeben werden kann, die sicherstellt, dass die Aufbereitung erfolgt, bevor es zu einer Patientengefährdung kommt.

Ein zweiter Aspekt der Erfindung betrifft ein Beatmungssystem. Das Beatmungssystem kann beispielsweise ein System zur Narkotisierung und/oder Beatmung sein. Das Beatmungssystem umfasst eine Vorrichtung, wie sie vor- und nachstehend beschrieben ist. Ferner kann das Beatmungssystem ein Beatmungsgerät umfassen, wobei die Vorrichtung über den ersten Anschluss an das Beatmungsgerät angeschlossen ist. Zusätzlich oder alternativ zum Beatmungsgerät kann das Beatmungssystem neben der Vorrichtung ein Atemgasleitungssystem zum Leiten von Atemgas hin zu und/oder weg von einem Beatmungsgerät umfassen, wobei die Vorrichtung über den zweiten Anschluss an das Atemgasleitungssystem angeschlossen ist.

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann der dritte Leitungsabschnitt einen zum ersten Leitungsabschnitt hin abfallenden ersten Teilabschnitt und/oder einen zum zweiten Leitungsabschnitt hin abfallenden zweiten Teilabschnitt umfassen, sodass Kondensat aufgrund der Schwerkraft in mindestens einer Richtung aus dem dritten Leitungsabschnitt abfließen kann.

Anders ausgedrückt kann der erste bzw. zweite Teilabschnitt in seiner Längsrichtung betrachtet im betriebsfähigen Zustand der Vorrichtung eine bestimmte Neigung zur Horizontalen haben. Dabei kann ein erstes Ende des ersten Teilabschnitts in vertikaler Richtung betrachtet (deutlich) höher als ein zweites Ende des ersten Teilabschnitts liegen, beispielsweise mindestens 1 cm, mindestens 10 cm, mindestens 50 cm oder mindestens 100 cm höher. Das Gleiche kann in entsprechender Weise für den zweiten Teilabschnitt zutreffen. Zwischen seinem ersten Ende und seinem zweiten Ende kann der erste bzw. zweite Teilabschnitt zumindest teilweise geradlinig und/oder zumindest teilweise gekrümmt verlaufen. Es kann zweckmäßig sein, wenn der erste bzw. zweite Teilabschnitt kontinuierlich, d. h. ohne Unterbrechung, zum jeweiligen Leitungsabschnitt hin abfällt. Möglich sind aber auch Varianten mit Unterbrechung. Das erste (höhere) Ende des ersten Teilabschnitts kann mit dem ersten (höheren) Ende des zweiten Teilabschnitts in einem Übergangsabschnitt, wie er beispielsweise nachstehend näher beschrieben wird, fluidisch verbunden sein. Dabei kann das zweite (niedrigere) Ende des ersten Teilabschnitts mit dem ersten Leitungsabschnitt und das zweite (niedrigere) Ende des zweiten Teilabschnitts mit dem zweiten Leitungsabschnitt fluidisch verbunden sein.

In der Leitung wird das Atemgas geleitet, das das von einem Patienten kommende Exspirationsgas enthalten kann. Das Exspirationsgas enthält in der Regel eine gewisse Feuchtigkeit. Selbst nach einer zusätzlichen Entfeuchtung, beispielsweise durch einen chemischen Entfeuchter, kann das Atemgas noch eine gewisse Restfeuchte aufweisen. So kann es sein, dass sich Feuchtigkeit aus dem Atemgas an einer Innenfläche der Leitung in Form von Kondensat niederschlägt. Das Kondensat kann eine Grundlage für das Wachstum von Biofilmen bilden. Dadurch, dass das Kondensat aus dem dritten Leitungsabschnitt abfließen kann, wird der dritte Leitungsabschnitt an seiner Innenfläche trocken gehalten. Somit kann das Wachsen eines Biofilms an der Innenfläche verhindert oder zumindest gehemmt werden.

Gemäß einer Ausführungsform können der erste Teilabschnitt und der zweite Teilabschnitt in einem Übergangsabschnitt ineinander übergehen. Dabei können die beiden Teilabschnitte ausgehend vom Übergangsabschnitt zum jeweiligen Leitungsabschnitt hin abfallen. Alternativ oder zusätzlich kann der Übergangsabschnitt im betriebsfähigen Zustand der Vorrichtung den höchsten Punkt des dritten Leitungsabschnitts oder der Leitung bilden.

Unter "Übergangsabschnitt" kann allgemein eine (fluiddichte) Verbindung zweier Enden der beiden Teilabschnitte verstanden werden. Beispielsweise kann der Übergangsabschnitt rohrförmig ausgebildet sein und/oder durch eine Muffe und/oder durch eine stoffschlüssige Verbindung gebildet sein.

Beispielsweise kann es sich bei den beiden Teilabschnitten um zumindest teilweise gerade und/oder zumindest teilweise gekrümmte Rohre handeln, die mit jeweils einem ihrer Enden so miteinander verbunden sind, dass die resultierende Rohrkombination die Form eines umgedrehten V oder eines umgedrehten U hat. Der Übergangsabschnitt kann dabei die Spitze des umgedrehten V bzw. U umfassen.

Beispielsweise können die beiden Teilabschnitte im Übergangsabschnitt ineinander übergehen und dabei in einem ersten Winkel zueinander ausgerichtet sein. Der erste Winkel kann beispielsweise 160° oder weniger, 120° oder weniger, 90° oder weniger, 60° oder weniger oder 30° oder weniger betragen.

Durch die Ausbildung eines höchsten Punkts in dem Übergangsabschnitt kann vorteilhafterweise sichergestellt werden, dass Kondensat allein aufgrund der Schwerkraft in mindestens einer Richtung abfließen kann. Zudem kann dadurch verhindert werden, dass Kondensat aus anderen Abschnitten der Leitung in den dritten Leitungsabschnitt (entgegen der Schwerkraft) hineinfließen kann.

Gemäß einer Ausführungsform kann mindestens einer der beiden Teilabschnitte einen schrägen Abschnitt, dessen Längsrichtung im betriebsfähigen Zustand der Vorrichtung schräg zur Horizontalen verläuft, und/oder einen vertikalen Abschnitt, dessen Längsrichtung im betriebsfähigen Zustand der Vorrichtung vertikal verläuft, umfassen.

Unter "vertikaler Abschnitt" kann verstanden werden, dass dieser Abschnitt im betriebsfähigen Zustand der Vorrichtung in seiner Längsrichtung (idealerweise) vertikal zu den horizontal ausgerichteten ersten Teilabschnitt bzw. zweitem Teilabschnitt verlaufen kann. Der vertikale Abschnitt und der erste bzw. zweite Teilabschnitt können somit in einem dritten Winkel zueinander ausgerichtet sein, der beispielweise 90° beträgt. Abweichungen von dem 90°-Winkel von beispielsweise 1° oder mehr, 5° oder mehr oder 10° oder mehr können jedoch auch zweckmäßig sein.

Der schräge Abschnitt und der vertikale Abschnitt können beispielsweise unmittelbar ineinander übergehen. Möglich ist auch, dass der schräge Abschnitt und der vertikale Abschnitt über einen separaten Verbindungsabschnitt miteinander verbunden sind.

Gemäß einer Ausführungsform kann der vertikale Abschnitt an einem seiner Enden mit dem jeweiligen Leitungsabschnitt und an seinem anderen Ende mit dem schrägen Abschnitt verbunden sein.

Beispielsweise können die schrägen Abschnitte der beiden Teilabschnitte im Übergangsabschnitt an ihrem einen Ende ineinander übergehen und dabei im ersten Winkel zueinander ausgerichtet sein.

Die schrägen Abschnitte der beiden Teilabschnitte können an ihrem anderen Ende jeweils in den jeweiligen vertikalen Abschnitt übergehen. Der schräge Abschnitt und der vertikale Abschnitt können dabei jeweils in einem zweiten Winkel zueinander ausgerichtet sein. Der zweite Winkel kann beispielsweise 160° oder weniger, 120° oder weniger, 90° oder weniger, 60° oder weniger, 45° oder weniger oder 30° oder weniger betragen.

Gemäß einer Ausführungsform kann die Vorrichtung ferner eine Heizeinrichtung umfassen, die ausgebildet sein kann, um den dritten Leitungsabschnitt auf eine erste Temperatur zum Vermeiden von Kondensation im dritten Leitungsabschnitt und/oder auf eine zweite Temperatur zum Abtöten und/oder Inaktivieren von Keimen zu erwärmen.

Unter "Heizeinrichtung" kann eine Vorrichtung verstanden werden, die Wärme in oder an der Leitung erzeugen kann. Beispielsweise kann die Heizeinrichtung als Heizdraht, beispielsweise als Widerstandsdraht, ausgebildet sein, der entlang der Leitung verläuft und Wärme in das Innere der Leitung abstrahlen kann. Der Heizdraht kann beispielsweise an der Außenwand der Leitung aufliegen oder in das Material der Leitung integriert sein.

Die Heizeinrichtung kann aber auch als Heizschlauch, Heizstab, Heizpatrone, Wärmestrahler oder Widerstandsheizung ausgebildet sein. Die Heizeinrichtung kann beispielsweise mit einer Steuereinrichtung eines Beatmungsgerätes gekoppelt sein und somit über das Beatmungsgerät steuerbar sein.

Die erste und die zweite Temperatur können miteinander übereinstimmen oder deutlich voneinander abweichen. Im letzteren Fall kann die Heizeinrichtung beispielsweise ausgebildet sein, um den dritten Leitungsabschnitt abwechselnd auf die erste Temperatur und die zweite Temperatur zu erwärmen. Die Heizeinrichtung kann beispielsweise ausgebildet sein, um den dritten Leitungsabschnitt mithilfe einer geeigneten Regelung so zu erwärmen, dass sich dessen tatsächliche Temperatur der ersten bzw. zweiten Temperatur annähert. Die Heizeinrichtung kann aber auch ausgebildet sein, um den dritten Leitungsabschnitt mithilfe einer geeigneten Steuerung, d. h. ohne Rückführung der tatsächlichen Temperatur des dritten Leitungsabschnitts, zu erwärmen, beispielsweise in bestimmten Intervallen, sodass sich die durchschnittliche tatsächliche Temperatur des dritten Leitungsabschnitts über mehreren Erwärmungsintervallen der ersten bzw. zweiten Temperatur annähert. Denkbar ist, dass das Beatmungssystem ausgebildet ist, um die Heizeinrichtung abwechselnd ein- und auszuschalten.

Beispielsweise kann die erste Temperatur zwischen 30°C und 100°C liegen. Durch Erwärmen des dritten Leitungsabschnitts auf die erste Temperatur kann die Innenfläche der Leitung trocken sein, da eine Kondensation vermieden oder verhindert werden kann.

Beispielsweise kann die zweite Temperatur zwischen 60 °C und 160 °C liegen. Durch Erwärmen des dritten Leitungsabschnitts auf die zweite Temperatur kann die Leitung zumindest abschnittsweise thermisch desinfiziert werden. Bei Temperaturen über 70 °C können bereits viele Mikroorganismen abgetötet oder inaktiviert werden.

Gemäß einer Ausführungsform kann die Vorrichtung ferner eine Bestrahlungseinrichtung umfassen, die ausgebildet sein kann, um den dritten Leitungsabschnitt mit einer Strahlung zum Abtöten und/oder Inaktivieren von Keimen zu bestrahlen. Die Bestrahlungseinrichtung kann insbesondere ausgebildet sein, um den dritten Leitungsabschnitt mit ultravioletter Strahlung zu bestrahlen.

Unter "Bestrahlungseinrichtung" kann eine Vorrichtung verstanden werden, die eine (elektromagnetische) Strahlung generieren und auf oder in die Leitung leiten kann. Unter Bestrahlungseinrichtung kann insbesondere eine Vorrichtung verstanden werden, die ultraviolette Strahlung (UV-Strahlung) zur Desinfektion von Luft, Wasser oder Oberflächen generieren kann. Beispielsweise kann die Bestrahlungseinrichtung als UV-Strahler, UV-Leuchtdiode, Diodenlaser, Excimerlaser, Quarzlampe oder Quecksilberdampflampe ausgebildet sein.

Die von der Bestrahlungseinrichtung ausgesandte (UV-)Strahlung kann beispielsweise in einem Wellenlängenbereich zwischen 10 und 380 Nanometern liegen. Insbesondere kann es sich bei der Strahlung um UV-C-Strahlung mit einer Wellenlänge zwischen 100 und 280 Nanometern, bevorzugt zwischen 205 und 280 Nanometern, besonders bevorzugt von 254 Nanometern, handeln.

Die Bestrahlungseinrichtung kann innerhalb oder außerhalb des dritten Leitungsabschnitts angeordnet sein. Im ersten Fall kann beispielsweise eine Quecksilberdampflampe in der Leitung angeordnet sein und über eine Quecksilberentladung UV-Licht generieren. Beispielsweise kann auch ein Excimerlaser in der Leitung angeordnet sein, der Excimer-Moleküle einleitet, die beim Abregen UV-Licht freisetzen.

Im zweiten Fall kann es zweckmäßig sein, wenn der dritte Leitungsabschnitt zumindest bereichsweise aus einem für die jeweilige Strahlung, z. B. UV-Licht, durchlässigen Material, insbesondere Quarzglas, Fluorethylenpropylen (FEP) und/oder Polymethylmethacrylat (PMMA), gefertigt ist. Dadurch wird gewährleistet, dass die Strahlung in den dritten Leitungsabschnitt gelangen kann, sodass dort Keime abgetötet oder inaktiviert werden. In einer konkreten Ausführung kann der dritte Leitungsabschnitt beispielsweise ein Quarzfenster umfassen, durch das UV-Licht in das Leitungsinnere geleitet werden kann.

Gemäß einer Ausführungsform kann die Vorrichtung ferner eine Auffangeinrichtung umfassen, die ausgebildet sein kann, um Kondensat aus dem ersten Leitungsabschnitt und/oder dem zweiten Leitungsabschnitt aufzufangen und/oder abzuleiten, bevor es in den dritten Leitungsabschnitt gelangt.

Die Auffangeinrichtung kann beispielsweise einen Auffangbehälter zum Auffangen des Kondensats umfassen. Der Auffangbehälter kann im ersten Leitungsabschnitt und/oder im zweiten Leitungsabschnitt angeordnet sein. Möglich ist auch, dass mindestens ein erster Auffangbehälter im ersten Leitungsabschnitt und mindestens ein zweiter Auffangbehälter im zweiten Leitungsabschnitt (getrennt vom ersten Auffangbehälter) angeordnet ist. Unter "Auffangbehälter" kann beispielsweise eine Schale oder ein Tank verstanden werden.

Zusätzlich oder alternativ kann die Auffangeinrichtung eine Ableitung zum Ableiten des Kondensats aus dem dritten Leitungsabschnitt umfassen. Die Ableitung kann vom ersten Leitungsabschnitt und/oder vom zweiten Leitungsabschnitt abzweigen. Möglich ist auch, dass eine erste Ableitung vom ersten Leitungsabschnitt und eine zweite Ableitung vom zweiten Leitungsabschnitt (getrennt von der ersten Ableitung) abzweigt. Unter "Ableitung" kann beispielsweise ein (flexibler) Schlauch, ein (starres) Rohr oder eine Kombination aus beidem verstanden werden. Alternativ kann die Auffangeinrichtung statt der Ableitung einen entsprechenden Anschluss zum Anschließen der Ableitung bzw. statt der Ableitungen entsprechende Anschlüsse zum Anschließen der Ableitungen umfassen.

Gemäß einer Ausführungsform kann die Leitung zumindest im dritten Leitungsabschnitt zumindest teilweise aus einem Metall und/oder einem Polymermaterial, insbesondere aus Polyamid, gefertigt sein. Andere mögliche Materialien sind beispielsweise Polyetheretherketon (PEEK), Quarzglas, Fluorethylenpropylen (FEP) oder Polymethylmethacrylat (PMMA).

Solche Materialien können das Keimwachstum unterbinden und/oder hemmen, da sie Keimen kein Nährmedium bieten. Darüber können solche Materialien besonders hitze- und/oder UV-beständig sein. Die Verwendung von Quarzglas, Fluorethylenpropylen (FEP) oder Polymethylmethacrylat (PMMA) ermöglicht es beispielsweise zudem, den dritten Leitungsabschnitt in einfacher Weise so herzustellen, dass er für UV-Licht durchlässig ist. Dadurch kann die Leitung, zumindest im dritten Leitungsabschnitt, mit einer geeigneten Temperatur oder UV-Licht desinfiziert werden und dennoch langlebig ausgebildet sein.

Gemäß einer Ausführungsform kann die Leitung zumindest im dritten Leitungsabschnitt zumindest teilweise eine antiadhäsive und/oder antimikrobielle Innenfläche aufweisen.

Mithilfe einer antiadhäsiven Innenfläche kann die Adhäsion, also die Anhaftung von Keimen oder Biofilmen an der mit dem Atemgas in Berührung kommenden Innenseite der Leitung unterbunden oder zumindest (stark) gemindert werden. Eine solche antiadhäsive Innenfläche kann beispielsweise durch eine spezielle Oberflächenmodifikation auf molekularer und/oder struktureller Ebene realisiert sein. Beispielsweise kann die Innenfläche derart ausgebildet sein, dass sie hydrophobe Eigenschaften aufweist. Auf diese Weise kann eine unerwünschte Anlagerung von Wasser oder wässrigen Systemen wie beispielsweise Biofilmen effektiv verhindert werden. Ein ähnlicher Effekt kann mit einer speziell reibungsmindernden und/oder (hierarchisch) strukturierten Innenfläche erzielt werden. Vorteilhafter Weise wird die Benetzbarkeit der Innenfläche herabsetzt. Beispielsweise kann die Innenfläche eine mikro- und/oder nanoskopische Strukturierung mit Lotuseffekt aufweisen.

Mithilfe einer antimikrobiellen Innenfläche kann erreicht werden, dass Keime gezielt abgetötet oder inaktiviert werden oder dass das Wachstum von Keimen zumindest (stark) gehemmt wird. Somit kann der Bildung von Biofilmen im dritten Leitungsabschnitt (und darüber hinaus) entgegengewirkt werden.

Gemäß einer Ausführungsform kann die Innenfläche durch eine Schicht gebildet sein, die mindestens eines der folgenden Materialien umfassen kann: Silber, Kupfer, Zink, Platin oder deren Legierungen. In manchen Ausführungsformen kann die Leitung zumindest im dritten Leitungsabschnitt auch aus einem Kunststoff gefertigt sein, der mit antimikrobiellen Additiven versetzt wurde, beispielsweise mit Thiabendazol, Zinkpyrithion, Isothiazolinon oder 10,10'-Oxybisphenox-Arsin.

Eine Kombination verschiedener Materialien kann vorteilhaft sein, da die verschiedenen Materialien unterschiedliche Wirkungen aufweisen können. So haben beispielsweise Zink und Silber bereits in geringen Konzentrationen eine starke antibakterielle Wirkung, wirken jedoch erst in hohen Konzentrationen auch antimykotisch. Andere Wirkstoffe haben große antimykotische Wirkung bei geringerer antibakterieller Wirkung, beispielsweise Isothiazolinon und Thiabendazol.

Die Innenfläche kann somit antimikrobielle Substanzen umfassen und/oder an die Umgebung abgeben. Die antimikrobiellen Substanzen können das Wachstum von Keimen auf molekularer Ebene verhindern oder zumindest hemmen. Beispiele für solche antimikrobiellen Substanzen sind Silber, Kupfer oder Kupferlegierungen, da aus diesen Materialien Silber- bzw. Kupferionen freigesetzt werden können. Diese wirken keimabtötend.

Die voranstehend beschriebene Oberflächenbeschaffenheit und/oder Oberflächenbeschichtung kann somit durch Freisetzung von Wirkstoffen, die gegen die Keime wirken, aktiv den Aufbau von Biofilmen verhindern oder unterdrücken. Der Aufbau von Biofilmen kann darüber hinaus auch passiv verhindert oder unterdrückt werden, indem die Anheftung von Keimen oder Biofilmen erschwert oder verhindert wird.

Die Oberflächenbeschichtung kann beispielsweise durch chemische und/oder physikalische Gasphasenabscheidung hergestellt werden. Dies stellt eine besonders einfache Herstellung der Innenfläche dar.

Gemäß einer Ausführungsform kann die Vorrichtung ferner ein Ventil umfassen, das ausgebildet sein kann, um einen Atemgasstrom durch die Leitung vom ersten Anschluss zum zweiten Anschluss zu ermöglichen und vom zweiten Anschluss zum ersten Anschluss zu sperren.

Zu diesem Zwecke kann das Ventil als Rückschlagventil, insbesondere als mechanisches Rückschlagventil, ausgebildet sein. Dieses kann einfach oder belastet ausgeführt sein, beispielsweise als federbelastetes Rückschlagventil. Möglich ist aber auch ein elektrisch steuerbares (Magnet-)Ventil. Das Ventil ist ausgebildet, um einen Atemgasstrom vom Beatmungsgerät zum AGFS zu ermöglichen. Zudem ist das Ventil ausgebildet, um einen Atemgasstrom vom AGFS zum Beatmungsgerät zu sperren. Somit kann verhindert werden, dass möglicherweise kontaminiertes oder verschmutztes (Atem-)Gas vom Atemgasleitungssystem zurück ins Beatmungsgerät und weiter zum Patienten strömt.

Das Ventil kann im ersten Leitungsabschnitt oder im zweiten Leitungsabschnitt angeordnet sein. Beispielsweise kann das Ventil deutlich näher am zweiten Anschluss als am ersten Anschluss angeordnet sein. Dies ist so zu verstehen, dass ein Abstand des Ventils zum zweiten Anschluss beispielsweise nicht mehr als 70 %, nicht mehr als 50 %, nicht mehr als 20 %, nicht mehr als 10 % oder nicht mehr als 1 % eines Abstands des Ventils zum ersten Anschluss beträgt. Das Ventil kann auch unmittelbar an den zweiten Anschluss angrenzend angeordnet sein.

Eine derartige Anordnung des Ventils hat den Vorteil, dass potenziell mit Keimen belastetes Gas gar nicht oder nur in sehr geringen Mengen vom Atemgasleitungssystem in die Leitung gelangen kann. Somit kann eine Verkeimung in Richtung des Beatmungsgerätes wirksam unterbunden werden.

Gemäß einer Ausführungsform kann die Vorrichtung ferner einen Filter umfassen, der ausgebildet sein kann, um Keime aus dem durch die Leitung strömenden Atemgas zu filtern. Unter "filtern" kann beispielsweise eine (mechanische oder chemische) Abscheidung von Keimen aus dem Atemgas, eine (chemische oder biologische) Inaktivierung von Keimen oder eine Kombination aus beiden verstanden werden.

Gemäß einer Ausführungsform kann der Filter durch einen Schwebstofffilter und/oder einen chemischen Filter ausgebildet sein.

Der Filter kann beispielsweise ausgebildet sein, um Keime mithilfe eines geeigneten Filtermaterials mechanisch zurückzuhalten. Der Filter kann beispielsweise mindestens einen der folgenden Filtertypen umfassen: einen (Hochleistungs-)Partikelfilter, z. B. in Form eines EPA-Filters (EPA = *Efficient Particulate Air*); einen Schwebstofffilter, z. B. in Form eines HEPA-Filters (HEPA = *High-EfficiencyParticulate Air*); einen Hochleistungsschwebstofffilter, z. B. in Form eines ULPA-Filters (ULPA *= Ultra-Low Penetration Air*). Der Filter ist in bevorzugten Ausführungsformen ein HEPA-Filter.

Unter einem chemischen Filter kann eine Vorrichtung verstanden werden, die Keime chemisch inaktivieren und/oder absorbieren kann. Beispielsweise kann der chemische Filter mindestens eine der folgenden Vorrichtungen umfassen: einen Aktivkohlefilter; einen Ozonfilter, einen Plasmagenerator.

Der Aktivkohlefilter kann ausgebildet sein, um Keime zu absorbieren. Der Ozonfilter kann ausgebildet sein, um Ozon in die Leitung einzuleiten, um diese zu sterilisieren. Der Plasmagenerator kann ausgebildet sein, Atmosphärenplasma zu generieren und in die Leitung einzuleiten, um diese zu sterilisieren.

Der Filter kann beispielsweise im ersten Leitungsabschnitt und/oder im zweiten Leitungsabschnitt angeordnet sein. Beispielsweise kann der Filter deutlich näher am zweiten Anschluss als am ersten Anschluss angeordnet sein. Dies ist so zu verstehen, dass ein Abstand des Filters zum zweiten Anschluss beispielsweise nicht mehr als 70 %, nicht mehr als 50 %, nicht mehr als 20 %, nicht mehr als 10 % oder nicht mehr als 1 % eines Abstands des Filters zum ersten Anschluss beträgt. Der Filter kann auch unmittelbar an den zweiten Anschluss angrenzend angeordnet sein. Eine derartige Anordnung des Filters hat den Vorteil, dass potenziell mit Keimen belastetes Gas gar nicht oder nur in sehr geringen Mengen vom Atemgasleitungssystem in die Leitung gelangen kann. Somit kann eine Verkeimung in Richtung des Beatmungsgerätes wirksam unterbunden werden.

Beispielsweise kann der Filter in der Leitung zwischen dem Ventil und dem zweiten Anschluss angeordnet sein. Alternativ oder zusätzlich ist auch eine Anordnung zwischen dem Ventil und dem ersten Anschluss möglich.

In der Leitung können auch mehrere solcher Filter an verschiedenen Stellen angeordnet, beispielsweise miteinander in Reihe geschaltet sein. Dabei können die Filter in ihrem Filtertyp zumindest teilweise voneinander abweichen und/oder zumindest teilweise miteinander übereinstimmen. Auf diese Weise kann die Filterwirkung weiter verbessert werden.

Gemäß einer Ausführungsform kann die Leitung eine Gesamtlänge zwischen 2 cm und 100 cm, bevorzugt zwischen 3 cm und 70 cm, besonders bevorzugt zwischen 3 cm und 51 cm, weiter bevorzugt zwischen 3 cm und 11 cm haben. Gemäß einer Ausführungsform kann der dritte Leitungsabschnitt eine Länge zwischen 2 cm und 50 cm, bevorzugt zwischen 2 cm und 20 cm, besonders bevorzugt zwischen 2 cm und 5 cm haben. Gemäß einer Ausführungsform kann der dritte Leitungsabschnitt einen Durchmesser zwischen 5 mm und 50 mm, bevorzugt zwischen 8 mm und 40 mm, besonders bevorzugt zwischen 10 mm und 30 mm haben.

### Kurze Beschreibung der Figuren

Im Folgenden werden Ausführungsformen der Erfindung mit Bezug auf die beigefügten Figuren beschrieben. Die Erfindung ist nicht auf die dargestellten Ausführungsbeispiele beschränkt. Weder die Beschreibung noch die Figuren sind als Beschränkung des Umfangs der Erfindung zu verstehen.
**Figur 1** zeigt ein Beatmungssystem 100 gemäß einer Ausführungsform der Erfindung.
**Figur 2** zeigt eine Vorrichtung 20 gemäß einer ersten Ausführungsform der Erfindung.
**Figur 3** zeigt eine Vorrichtung 20 gemäß einer zweiten Ausführungsform der Erfindung.
**Figur 4** zeigt eine Vorrichtung 20 gemäß einer dritten Ausführungsform der Erfindung.

Die Figuren sind rein schematisch und nicht maßstabsgetreu. Werden in verschiedenen Figuren gleiche Bezugszeichen verwendet, so bezeichnen diese Bezugszeichen gleiche oder gleichwirkende Merkmale.

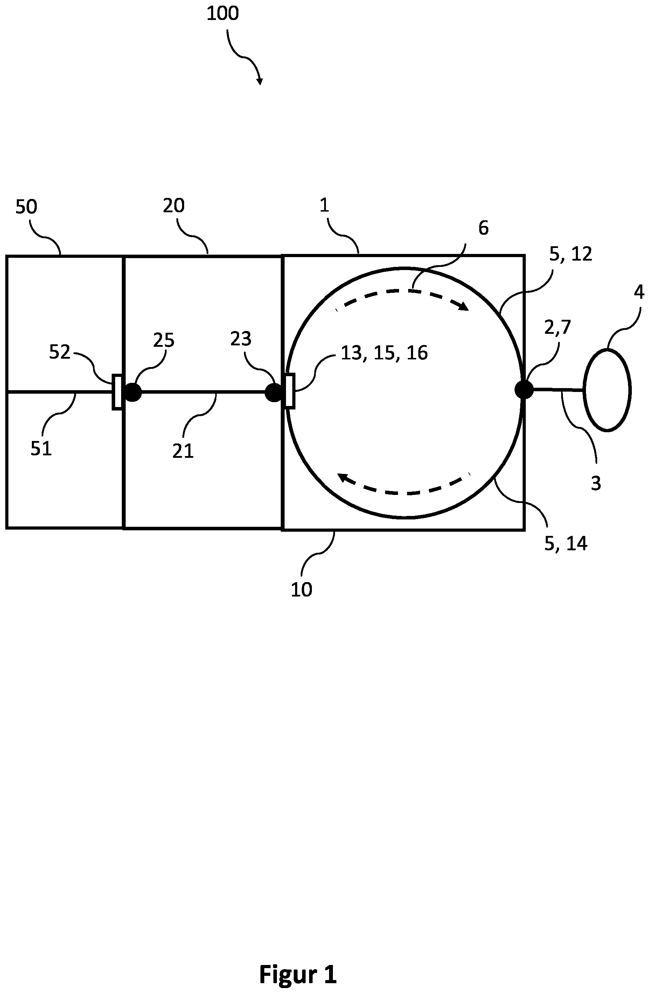

**Figur 1** zeigt ein Beatmungssystem 100 zur Narkotisierung und/oder zur Beatmung. Das Beatmungssystem 100 umfasst ein Beatmungsgerät 1. Alternativ oder zusätzlich kann das Beatmungssystem 100 ein Atemgasleitungssystem 50 zum Leiten von Atemgas hin zum und/oder weg vom Beatmungsgerät 1. Ferner umfasst das Beatmungssystem 100 eine Vorrichtung 20 zur Keimabwehr, wie sie vor- und nachstehend beschrieben ist.

Das Beatmungsgerät 1 umfasst eine Atemgasleitung 5 zum Leiten von Atemgas und zumindest einen Anschluss 2. Das Beatmungsgerät 1 umfasst in der Regel ein Gehäuse 10, in dem die Atemgasleitung 5 zumindest teilweise angeordnet sein kann.

Der Anschluss 2 kann beispielsweise am Gehäuse 10 angeordnet sein und einen Atemgasausgang und/oder Atemgaseinlass darstellen. Über den Anschluss 2 kann ein Patienteninterface 4 an das Beatmungsgerät 1 angeschlossen werden, über das ein hier nicht dargestelltes Lebewesen mit einem Atemgas versorgt werden kann. An den Anschluss 2 kann das Patienteninterface 4 direkt angeschlossen werden (nicht gezeigt). An den Anschluss 2 kann auch ein Schlauch oder Schlauchsystem 3 angeschlossen werden. Der Schlauch oder das Schlauchsystem 3 kann das Patienteninterface 4 mit dem Beatmungsgerät 1 atemgasleitend verbinden.

Im oder am Anschluss 2 kann ein Filter 7 oder ein Filtersystem aus mehreren Filtern angeordnet sein. Der Filter 7 kann ein Partikel- und/oder Schwebstofffilter sein. Der Filter 7 kann austauschbar sein. Der Filter 7 kann Schwebstoffe und insbesondere auch Keime aus dem Atemgas abscheiden. Der Filter 7 kann ausgewählt sein aus der Gruppe: Hochleistungs-Partikelfilter (EPA-Filter); Schwebstofffilter (HEPA-Filter); Hochleistungs-Schwebstofffilter (ULPA-Filter). In bevorzugten Ausführungsformen ist der Filter mindestens ein HEPA-Filter.

Die Atemgasleitung 5 kann zumindest teilweise innerhalb des Gehäuses 10 ausgebildet sein. Die Atemgasleitung 5 kann auch zumindest teilweise außerhalb des Gehäuses 10 ausgebildet sein, beispielsweise in dem Schlauchsystem 3. Die Atemgasleitung 5 kann einen inspiratorischen Zweig 12 umfassen. Alternativ oder zusätzlich kann die Atemgasleitung 5 einen exspiratorischen Zweig 14 umfassen.

In manchen Ausführungsformen können der inspiratorische Zweig 12 und der exspiratorische Zweig 14 in einer Übergangsleitung 13 ineinander übergehen. Die Übergangsleitung 13 kann atemgasleitend zwischen dem inspiratorischen Zweig 12 und dem exspiratorischen Zweig 14 ausgebildet sein. Der inspiratorische Zweig 12 kann sodann atemgasleitend zwischen der Übergangsleitung 13 und dem Anschluss 2 ausgebildet sein. Der exspiratorische Zweig 14 kann atemgasleitend zwischen dem Anschluss 2 und der Übergangsleitung 13 ausgebildet sein.

Über einen hier nicht dargestellten Atemgasantrieb und/oder Ventile in der Atemgasleitung 5 kann eine Strömungsrichtung 6 des Atemgases vorgegeben werden, die in den Figuren als gestrichelter Pfeil dargestellt ist. Die Strömungsrichtung 6 verläuft im inspiratorischen Zweig 12 in der Regel im Wesentlichen von der Übergangsleitung 13 zum Anschluss 2. Die Strömungsrichtung 6 verläuft im exspiratorischen Zweig 14 in der Regel im Wesentlichen vom Anschluss 2 zu der Übergangsleitung 13.

Der Schlauch 3 kann zur Inspiration und/oder zur Exspiration dienen. In manchen Ausführungsformen kann auch ein Schlauchsystem 3 mit mindestens einem Inspirationsschlauch sowie mindestens einen Exspirationsschlauch angeschlossen werden (nicht gezeigt). In diesem Falle kann der inspiratorische Zweig 12 mit dem Inspirationsschlauch verbunden sein und der Exspirationsschlauch mit dem exspiratorischen Zweig 14.

Somit kann der Patient über den Inspirationsschlauch mit Atemgas versorgt werden und über den Exspirationsschlauch ausatmen. Das Exspirationsgas kann über den Exspirationsschlauch in das Beatmungsgerät 1 - genauer in den exspiratorischen Zweig 14 - rückgeführt werden. Über eine Rückführung des Exspirationsgases in das Beatmungsgerät 1 kann ein Kreislauf des Atemgases realisiert sein. Das Exspirationsgas kann wieder für die Inspiration verwendet wird. Bei Verwendung eines Zweischlauchsystems kann der Anschluss 2 als Y-Stück ausgebildet sein, über das der Inspirationsschlauch und der Exspirationsschlauch verbindbar sind.

Bei der Führung des Atemgases in einem Kreislauf kann dem Exspirationsgas Kohlendioxid CO2 entzogen werden. Hierfür kann in der Atemgasleitung 5 ein hier nicht dargestellter CO2-Absorber angeordnet sein. Ferner kann dem Exspirationsgas Feuchtigkeit entzogen werden. Hierfür kann in der Atemgasleitung 5 ein hier nicht dargestellter Entfeuchter angeordnet sein. Zudem kann dem Atemgas bedarfsabhängig Frischgas und/oder Sauerstoff und/oder Anästhetika zugeführt werden.

Das Beatmungsgerät kann einen Atemgasauslass 15 umfassen. Der Atemgasauslass 15 kann in oder an der Übergangsleitung 13 angeordnet sein. Der Atemgasauslass 15 kann innerhalb des Gehäuses 10 angeordnet sein. Der Atemgasauslass 15 kann auch derart angeordnet sein, dass er im oder am Gehäuse 10 des Beatmungsgerätes angeordnet ist und einen Ausgang aus diesem darstellt.

Über den Atemgasauslass 15 kann Atemgas zumindest zeitweise, zumindest teilweise aus der Atemgasleitung 5 ableitbar sein. Durch Ableitung eines Teilgases aus dem Kreislauf kann der Druck während der Inspiration limitiert werden. Zudem kann über die Ableitung eines Teilgases gewährleistet werden, dass ein Gasaustausch stattfindet. Über den Gasaustausch können Metabolite der Lunge (wie beispielsweise Methan oder Ammoniak) aus dem Kreislauf entfernt werden. Frischgas und/oder Sauerstoff und/oder Anästhetika können dem Kreislauf bedarfsabhängig zugeführt werden.

Zu diesem Zwecke kann im oder am Atemgasauslass 15 ein Ventil 16 angeordnet sein. Das Ventil 16 kann ausgebildet sein, um Atemgas aus der Atemgasleitung 5 abzuleiten. Das Ventil 16 kann beispielsweise als steuerbares Überdruckventil (APL-Ventil) ausgebildet sein, um einen Inspirationsdruck zu regeln. Das Ventil 16 kann ausgebildet sein, um Atemgas aus der Atemgasleitung 5 abzuleiten, wenn der Druck in der Atemgasleitung 5 einen gewünschten, voreingestellten inspiratorischen Druck übersteigt. Das Ventil 16 kann ausgebildet sein, um das Atemgas vollständig aus dem Gaskreislauf 5 abzuleiten. Das Ventil 16 kann bevorzugt ausgebildet sein, um das Atemgas nur teilweise aus dem Gaskreislauf abzuleiten.

Da das Beatmungssystem 100 auch ausgebildet ist, einen Patienten zu narkotisieren, kann das abzuführende Atemgas neben dem normalen Atemgas auch potentiell gesundheits- und/oder umweltschädliche Substanzen enthalten wie beispielsweise volatile Anästhetika oder Metabolite der Lunge. Aus diesem Grund kann das Atemgas in vorteilhaften Ausführungsformen kontrolliert abgeleitet und entsorgt werden.

Zu diesem Zwecke kann das Beatmungssystem 100 ein Atemgasleitungssystem 50 zum kontrollierten Leiten von Atemgas umfassen. Das Atemgasleitungssystem 50 kann ausgebildet sein, Atemgas hin zum Beatmungsgerät 1 zu leiten. Insbesondere kann das Atemgasleitungssystem 50 auch ausgebildet sein, Atemgas weg vom Beatmungsgerät 1 zu leiten. Insbesondere können über das Atemgasleitungssystem 50 überschüssige Atemgase oder Gasgemische aus einem zu einem Patienten führenden Gaskreislauf entnommen werden. Über das Atemgasleitungssystem 50 können potenziell umwelt- oder gesundheitsschädliche Stoffe kontrolliert entnommen, aufbereitet und/oder entsorgt werden. Zu diesem Zweck umfasst das Atemgasleitungssystem 50 eine Atemgasleitung 51 zur Leitung vom Atemgas. Ferner umfasst das Atemgasleitungssystem 50 einen Atemgaseinlass 52 zur Kopplung der Atemgasleitung 51 mit dem Atemgasauslass 15 des Beatmungsgerätes 1.

Das Beatmungssystem 100 umfasst ferner eine Vorrichtung 20 zur Keimabwehr. Gemäß einer Ausführungsform der Erfindung ist die Vorrichtung 20 zwischen dem Beatmungsgerät 1 und dem Atemgasleitungssystem 50 angeordnet, um diese beiden Komponenten vor einer gegenseitigen Verunreinigung mit Keimen zu schützen. Die Vorrichtung 20 ist als Keimbarriere ausgebildet, die verhindert, dass Keime vom Beatmungsgerät 1 in das Atemgasleitungssystem 50 gelangen können. Insbesondere verhindert die Vorrichtung 20 auch, dass Keime vom Atemgasleitungssystem 50 in das Beatmungsgerät 1 gelangen können.

Die Vorrichtung 20 umfasst eine Leitung 21 zum Leiten des Atemgases. Die Leitung 21 kann zwischen dem Beatmungsgerät 1 und dem Atemgasleitungssystem 50 angeordnet sein. Die Leitung 21 umfasst einen ersten Anschluss 23 zum Anschließen der Leitung 21 an das Beatmungsgerät. Ferner umfasst die Leitung 21 einen zweiten Anschluss 25 zum Anschließen der Leitung 21 an das Atemgasleitungssystem 50.

Die Vorrichtung 20 kann über den ersten Anschluss 23 mit dem Beatmungsgerät 1 koppelbar sein. Insbesondere kann der erste Anschluss 23 mit dem Atemgasauslass 15 des Beatmungsgeräts 1 koppelbar sein. Alternativ oder zusätzlich kann die Vorrichtung 20 über den zweiten Anschluss 25 mit dem Atemgasleitungssystem 50 koppelbar sein. Insbesondere kann der zweiten Anschluss 25 mit dem Atemgaseinlass 52 des Atemgasleitungssystem 50 koppelbar sein. Aus der Figur 1 wird ersichtlich, dass die Leitung 21 zwischen dem Atemgasauslass 15 des Beatmungsgerätes 1 und dem Atemgaseinlass 52 der Atemgasleitungssystems 50 angeordnet sein kann.

Die Vorrichtung 20 kann in manchen Ausführungsformen eine geschlossene Einheit sein, die separat, beispielsweise mit einem eigenen Gehäuse, ausgebildet ist. Sodann kann die Vorrichtung 20 über die beiden Anschlüsse 23,25 mit den anderen Komponenten des Systems, dem Beatmungsgerät 1 und dem Atemgasleitungssystem 50, gekoppelt werden.

Die Vorrichtung 20 kann in manchen Ausführungsformen auch im Gehäuse 10 des Beatmungsgerätes 1 untergebracht sein, beispielsweise fest verbaut oder herausnehmbar. Die Vorrichtung 20 kann bevorzug ausgebildet sein, dass sie separat aus dem Beatmungsgerät 1 entnommen werden kann.

Die Vorrichtung 20 kann somit als Nachrüstset konstruiert sein, das in bestehende Beatmungssysteme oder Beatmungsgeräte eingefügt werden kann. Die Vorrichtung 20 kann beispielsweise einfach als kleiner Kasten ausgebildet sein, den man an das Beatmungsgerät anschließen kann. Die Abmessungen können beispielsweise in einem Bereich zwischen 3x3x3 cm bis 10x10x10 cm liegen. Die Vorrichtung 20 sollte mechanisch stabil, gasdicht und passend am Gerät anzubringen sein. Zudem sollte ein elektrischer Anschluss gegeben sein, da für die Heizeinrichtung Strom vorhanden sein muss und es einen Regelkreis braucht, um die Temperatur zu halten. Somit kann die Vorrichtung 20 (bzw. das Nachrüstset) auch eine Steuerelektronik und/oder einen elektrischen Anschluss und/oder eine Anzeigeeinrichtung umfassen.

In einer konkreten Ausführungsform kann die Vorrichtung 20 beispielsweise als Edelstahlblock ausgeführt sein. Dieser Edelstahlblock kann mit einem zweiten Edelstahlblock in Verbindung gebracht werden, der im Beatmungsgerät verbaut sein kann und eine Heizpatrone umfasst. Derart ist eine besonders einfache Wärmeübertragung auf die Vorrichtung 20 gegeben.

Die Leitung 21 kann beispielsweise aus einem Metall oder aus einem Polymermaterial oder aus einer Kombination der beiden vorgenannten Materialien gefertigt sein. Ferner kann die Leitung 21 zumindest abschnittsweise eine antiadhäsive und/oder antimikrobielle Innenfläche aufweisen. Die Innenfläche kann hierbei beispielsweise durch eine Silber- oder Kupfer-Schicht gebildet sein.

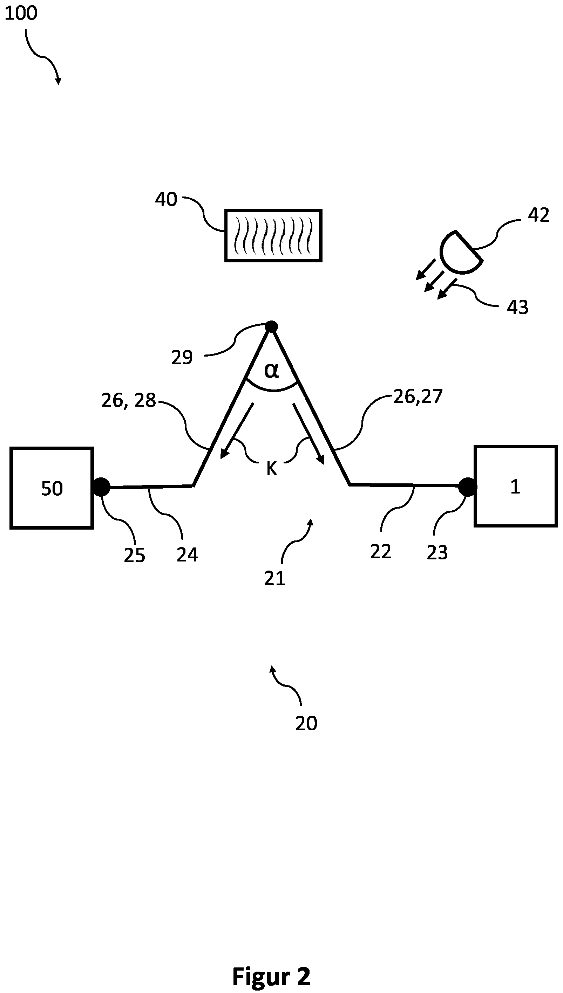

Die Figuren 2 bis 4 zeigen Ausführungsformen der Erfindung, wobei die räumliche Darstellung sehr schematisch ist, aber grundsätzlich derjenigen entspricht, in der sich die Vorrichtung 20 in einem betriebsfähigen Zustand befindet. Das bedeutet, dass die Schwerkraft vom oberen Blattrand in Richtung zum unteren Blattrand wirkt.

**Figur 2** zeigt die Vorrichtung 20 gemäß einer ersten Ausführungsform der Erfindung. Die Leitung 21 umfasst den ersten Leitungsabschnitt 22 mit dem ersten Anschluss 23. Über den ersten Anschluss 23 ist die Leitung 21 an das Beatmungsgerät 1 anschließbar. Die Leitung 21 umfasst den zweiten Leitungsabschnitt 24 mit dem zweiten Anschluss 25. Über den zweiten Anschluss 25 ist die Leitung 21 an das Atemgasleitungssystem 50 anschließbar. Ferner umfasst die Leitung 21 einen dritten Leitungsabschnitt 26. Über den dritten Leitungsabschnitt 26 sind der erste Leitungsabschnitt 22 und der zweite Leitungsabschnitt 24 miteinander verbunden.

Die unterschiedlichen vor- und nachstehend beschriebenen Abschnitte der Leitung 21 stehen in einer fluidischen Verbindung. Die Enden zweier Abschnitte können jeweils derart miteinander verbunden sein, dass die Leitung 21 ein durchgehendes Lumen aufweist, in dem das Atemgas bevorzugt störungsfrei geführt werden kann. Die Verbindung ist beispielsweise stoffschlüssig ausgeführt.

Der dritte Leitungsabschnitt 26 ist als Keimbarriere ausgebildet, um zu verhindern, dass Keime aus dem ersten Leitungsabschnitt 22 in den zweiten Leitungsabschnitt 24 und/oder aus dem zweiten Leitungsabschnitt 24 in den ersten Leitungsabschnitt 22 gelangen.

Die Leitung 21 kann eine Gesamtlänge zwischen 2 cm und 100 cm, bevorzugt zwischen 3 cm und 70 cm, besonders bevorzugt zwischen 3 cm und 51 cm, weiter bevorzugt zwischen 3 cm und 11 cm haben. Der dritte Leitungsabschnitt 26 kann eine Länge zwischen 2 cm und 50 cm, bevorzugt zwischen 2 cm und 20 cm, besonders bevorzugt zwischen 2 cm und 5 cm haben. Der dritte Leitungsabschnitt 26 kann einen Durchmesser zwischen 5 mm und 50 mm, bevorzugt zwischen 8 mm und 40 mm, besonders bevorzugt zwischen 10 mm und 30 mm haben.

Aus den Figuren 2 bis 4 ist ersichtlich, dass der dritte Leitungsabschnitt 26 einen zum ersten Leitungsabschnitt 22 hin abfallenden ersten Teilabschnitt 27 umfassen kann. Ferner ist gezeigt, dass der dritte Leitungsabschnitt 26 zudem einen zum zweiten Leitungsabschnitt 24 hin abfallenden zweiten Teilabschnitt 28 umfassen kann. In einem noch einfacheren Ausführungsbeispiel kann es auch zweckdienlich sein, dass nur der erste Teilabschnitt 27 oder nur der zweite Teilabschnitt 28 zum jeweiligen Leitungsabschnitt 22, 24 hin abfallend ausgebildet ist (nicht gezeigt).

Potenziell anfallendes Kondensat K kann durch die abfallenden Teilabschnitte 27,28 aufgrund der Schwerkraft in mindestens einer Richtung aus dem dritten Leitungsabschnitt 26 abfließen. Die Abflussrichtung des Kondensats K ist in der Figur 2 beispielhaft mit einem Pfeil dargestellt.

Die Leitung 21 weist in Längsrichtung eine längste Ausdehnung auf. Im betriebsfähigen Zustand kann die Leitung 21 in seiner Längsrichtung betrachtet zumindest teilweise waagerecht angeordnet sein. Aus den Figuren wird ersichtlich, dass beispielsweise der erste Leitungsabschnitt 22 und der zweite Leitungsabschnitt 24 waagerecht angeordnet sein können. Der dritte Leitungsabschnitt 26 ist dahingegen zumindest teilweise nicht waagerecht angeordnet. In den Ausführungsbeispiel gemäß der Figuren weisen der erste Teilabschnitt 27 und der zweite Teilabschnitt 28 zumindest teilweise eine Neigung zur Horizontalen auf.

Diejenigen Enden der Teilabschnitte 26,27, die mit dem jeweiligen ersten Leitungsabschnitt 22 oder zweiten Leitungsabschnitt 24 verbunden sind, liegen in vertikaler Richtung betrachtet deutlich tiefer als die jeweiligen gegenüberliegenden Enden.

An den gegenüberliegenden Enden können der erste Teilabschnitt 27 und der zweite Teilabschnitt 28 in einem Übergangsabschnitt 29 ineinander übergehen. Dieser liegt in vertikaler Richtung deutlich höher als die jeweiligen gegenüberliegenden Enden des ersten Teilabschnitts 27 und des zweiten Teilabschnitts 28. Durch die dadurch entstehende Neigung ist eine Abflussrichtung des Kondensats K vorgegeben.

In den Figuren 2 bis 4 ist gezeigt, dass der erste Teilabschnitt 27 und der zweite Teilabschnitt 28 ausgehend vom Übergangsabschnitt 29 zum jeweiligen Leitungsabschnitt 22, 24 hin abfallen können. Der Übergangsabschnitt 29 bildet sodann im betriebsfähigen Zustand der Vorrichtung 20 den höchsten Punkt des dritten Leitungsabschnitts 26 bzw. der Leitung 21.

In den Ausführungsbeispielen der Figuren 2 bis 4 sind der erste Teilabschnitt 27 und der zweite Teilabschnitt 28 spiegelsymmetrisch ausgebildet. Entgegen der Darstellung in den Figuren kann die Leitung 21 bzw. der dritte Leitungsabschnitt 26 auch asymmetrisch ausgebildet sein, beispielsweise in Form einer Kombination der Ausführungen gemäß der Figuren 2, 3 oder 4.

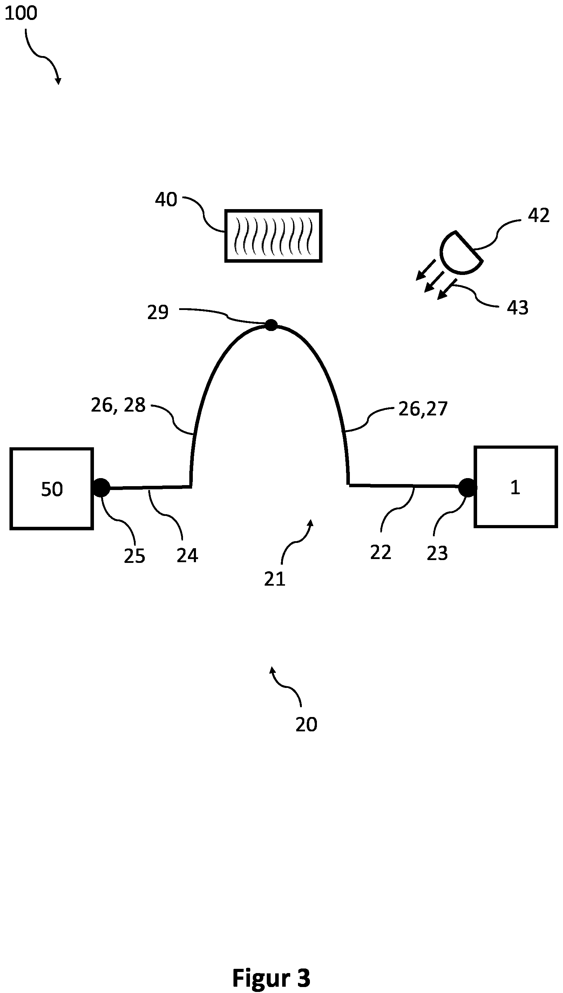

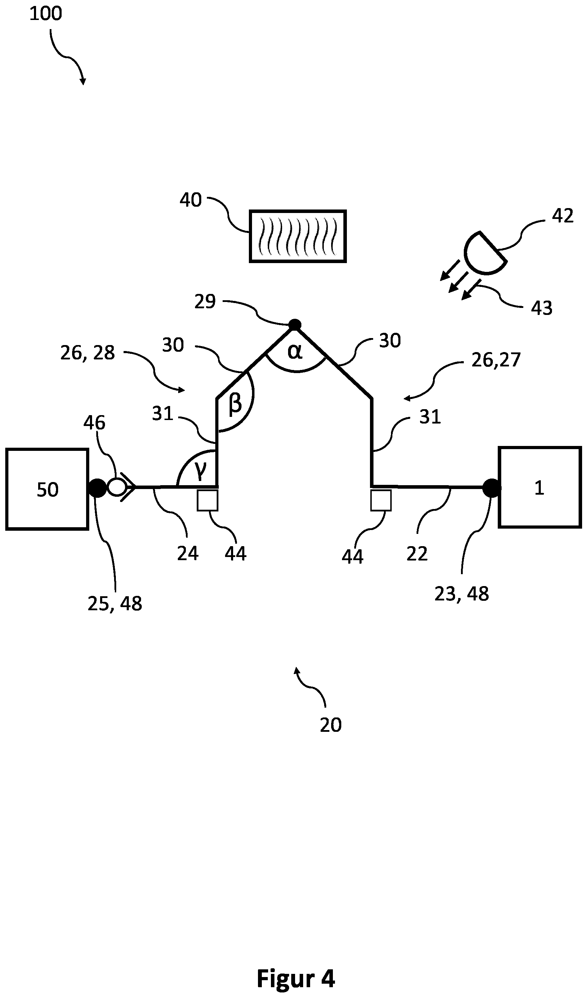

Figur 2 zeigt, dass die Abschnitte 27, 28 geradlinig verlaufen und die Neigung kontinuierlich sein kann. Der erste Teilabschnitt 27 und der zweite Teilabschnitt 28 gehen in diesem Beispiel an jeweils einem ihrer Enden im Übergangsabschnitt 29 ineinander über. Dort können sie in einem ersten Winkel α gewinkelt zueinander stehen. Der erste Winkel α kann beispielsweise 60° betragen. Die resultierende Formgebung des dritten Leitungsabschnitts 26 kann somit insgesamt die Form eines umgedrehten "V" aufweisen. Der Übergangsabschnitt 29 bildet dabei die Spitze des umgedrehten V. Andere Formen sind auch denkbar und beispielhaft in den Figuren 3 und 4 gezeigt.

**Figur 3** zeigt die Vorrichtung 20 gemäß einer zweiten Ausführungsform der Erfindung. In Figur 3 sind der erste Teilabschnitt 27 und der zweite Teilabschnitt 28 jeweils gekrümmt ausgebildet. Die resultierende Formgebung des dritten Leitungsabschnittes 26 weist somit insgesamt die Form eines umgedrehten "U" auf. Der Übergangsabschnitt 29 bildet dabei den Grund des umgedrehten U.

**Figur 4** zeigt die Vorrichtung 20 gemäß einer dritten Ausführungsform der Erfindung. In Figur 4 sind der erste Teilabschnitt 27 und der zweite Teilabschnitt 28 jeweils zumindest teilweise gerade ausgebildet, jedoch mit verschiedenen Abschnitten 30,31 unterschiedlicher Orientierung. Eine gekrümmte Ausbildung der Abschnitte 30,31 ist auch möglich (nicht gezeigt).

In dem Beispiel gemäß Figur 4 ist gezeigt, dass mindestens einer der beiden Teilabschnitte 27 ,28 einen schrägen Abschnitt 30, dessen Längsrichtung im betriebsfähigen Zustand der Vorrichtung 20 schräg zur Horizontalen verläuft, aufweisen kann. Alternativ oder zusätzlich kann mindestens einer der beiden Teilabschnitte 27 ,28 einen vertikalen Abschnitt 31, dessen Längsrichtung im betriebsfähigen Zustand der Vorrichtung 20 vertikal verläuft, aufweisen.

Aus Figur 4 wird ersichtlich, dass die vertikalen Abschnitte 31 an einem ihrer Enden mit dem jeweiligen Leitungsabschnitt 22,24 und an ihrem anderen Ende mit dem jeweiligen schrägen Abschnitt 31 verbunden sein können. In dem Beispiel gemäß Figur 4 sind die beiden Teilabschnitte 27, 28 spiegelsymmetrisch ausgebildet. Eine asymmetrische Ausführung ist auch denkbar (nicht gezeigt).

Figur 4 zeigt beispielhaft, dass die vertikalen Abschnitte 31 an einem ihrer Enden mit dem jeweiligen ersten Leitungsabschnitt 22 oder zweiten Leitungsabschnitt 24 verbunden sind. Dabei stehen der vertikale Abschnitt 31 und der erste Leitungsabschnitt 22 bzw. der zweite Leitungsabschnitt 24 in einem dritten Winkel γ zueinander, der beispielsweise 90° beträgt.

An ihrem anderen Ende können die vertikalen Abschnitte 31 jeweils mit einem Ende der schrägen Abschnitte 30 verbunden sein. Dort kann der vertikale Abschnitt 31 und der schräge Abschnitt 30 jeweils in einem zweiten Winkel β gewinkelt zueinander stehen. Der zweite Winkel β kann beispielsweise 45° betragen.

An ihrem anderen Ende können die schrägen Abschnitte 30 im Übergangsabschnitt 29 jeweils ineinander übergehen. Dort können sie im ersten Winkel α gewinkelt zueinander stehen. Der erste Winkel α kann beispielsweise 90° betragen.

Wie in den Figuren 2 bis 4 gezeigt, kann die Vorrichtung 20 eine Heizeinrichtung 40 umfassen. Mit der Heizeinrichtung 40, die beispielsweise als ein die Leitung 21 bzw. den dritten Leitungsabschnitt 26 umgebener Heizdraht ausgebildet sein kann, kann der dritte Leitungsabschnitt 26 auf eine erste Temperatur erwärmt werden, um eine Kondensation im dritten Leitungsabschnitt 26 zu vermeiden. Alternativ oder zusätzlich kann die Heizeinrichtung 40 den dritten Leitungsabschnitt 26 auf eine zweite Temperatur erwärmen, bei der Keime abgetötet und/oder inaktiviert werden.

Ferner kann die Vorrichtung 20 eine Bestrahlungseinrichtung 42 umfassen, die ausgebildet sein kann, um den dritten Leitungsabschnitt 26 mit einer Strahlung 43 zu bestrahlen, bei der Keime abgetötet und/oder inaktiviert werden. Beispielsweise kann die Bestrahlungseinrichtung 42 als UV-Strahler ausgebildet sein, um den dritten Leitungsabschnitt 26 mit ultravioletter Strahlung 43 zu bestrahlen.

Wie in Figur 4 exemplarisch gezeigt, kann die Vorrichtung 20 mindestens eine Auffangeinrichtung 44 umfassen, die ausgebildet sein kann, um Kondensat K aus dem ersten Leitungsabschnitt 22 und/oder dem zweiten Leitungsabschnitt 24 aufzufangen und/oder abzuleiten, bevor es in den dritten Leitungsabschnitt 26 gelangt. Die Auffangeinrichtung 44 kann beispielsweise als Auffangbehälter an dem ersten Leitungsabschnitt 22 angeordnet sein. Die Auffangeinrichtung 44 kann zudem auch an dem zweiten Leitungsabschnitt 24 eingerichtet sein.

Wie in Figur 4 exemplarisch gezeigt, kann die Vorrichtung 20 ferner ein Ventil 46 umfassen. Das Ventil 46 kann ausgebildet sein, um einen Atemgasstrom durch die Leitung 21 vom ersten Anschluss 23 zum zweiten Anschluss 25 zu ermöglichen und vom zweiten Anschluss 25 zum ersten Anschluss 23 zu sperren. Das Ventil 46 kann beispielsweise als (federbelastetes) Rückschlagventil ausgebildet sein. Beispielsweise kann das Ventil 46 unmittelbar an den zweiten Anschluss 25 angrenzend angeordnet sein. Somit kann verhindert werden, dass Gas vom Atemgasleitungssystem 50 in die Leitung 21 oder in das Beatmungsgerät 1 strömt.

Wie aus Figur 4 hervorgeht, kann in der Vorrichtung 20 mindestens ein Filter 48 angeordnet sein, beispielsweise ein Schwebstofffilter. Durch den Filter 48 können Keime aus dem durch die Leitung 21 strömenden Atemgas gefiltert werden. Der Filter kann beispielsweise im zweiten Leitungsabschnitt 24 unmittelbar an den zweiten Anschluss 25 angrenzend angeordnet sein. Beispielsweise kann der Filter 48 zwischen dem zweiten Anschluss 25 und dem Ventil 46 angeordnet sein. Der Filter 48 kann potenziell aus dem Atemgasleitungssystem 50 kommende Keime zurückhalten, bevor diese in die Leitung 21 gelangen können.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Einzelmerkmale mit ein.

### Bezugszeichenliste

- 1: Beatmungsgerät
- 2: Anschluss
- 3: Schlauch / Schlauchsystem
- 4: Patientenschnittstelle
- 5: Atemgasleitung
- 6: Strömungsrichtung des Atemgases
- 7: Filter
- 10: Gehäuse
- 12: Inspiratorischer Zweig
- 13: Übergangsleitung
- 14: Exspiratorischer Zweig
- 15: Atemgasauslass
- 16: Ventil
- 20: Vorrichtung zur Keimabwehr
- 21: Leitung
- 22: Erster Leitungsabschnitt
- 23: Erster Anschluss
- 24: Zweiter Leitungsabschnitt
- 25: Zweiter Anschluss
- 26: Dritter Leitungsabschnitt
- 27: Erster Teilabschnitt
- 28: Zweiter Teilabschnitt
- 29: Übergangsabschnitt
- 30: Schräger Abschnitt
- 31: Vertikaler Abschnitt
- 40: Heizeinrichtung
- 42: Bestrahlungseinrichtung
- 43: Strahlung
- 44: Auffangeinrichtung
- 46: Ventil
- 48: Filter
- 50: Atemgasleitungs-System (AGS)
- 51: Atemgasleitung
- 52: Atemgaseinlass
- 100: Beatmungssystem
- K: Kondensat
- α, β, γ: Erster / Zweiter / Dritter Winkel

## Patentansprüche

1. Vorrichtung (20) zur Keimabwehr in einem Beatmungssystem (100), wobei das Beatmungssystem (100) neben der Vorrichtung (20) ein Beatmungsgerät (1) und ein Atemgasleitungssystem (50) zum Leiten von Atemgas hin zum und/oder weg vom Beatmungsgerät (1) umfasst, wobei die Vorrichtung (20) eine Leitung (21) zum Leiten des Atemgases zwischen dem Beatmungsgerät (1) und dem Atemgasleitungssystem (50) umfasst, wobei die Leitung (21) einen ersten Leitungsabschnitt (22) mit einem ersten Anschluss (23) zum Anschließen der Leitung (21) an das Beatmungsgerät (1), einen zweiten Leitungsabschnitt (24) mit einem zweiten Anschluss (25) zum Anschließen der Leitung (21) an das Atemgasleitungssystem (50) und einen den ersten Leitungsabschnitt (22) mit dem zweiten Leitungsabschnitt (24) verbindenden dritten Leitungsabschnitt (26) umfasst, wobei der dritte Leitungsabschnitt (26) als Keimbarriere ausgebildet ist, um zu verhindern, dass Keime aus dem ersten Leitungsabschnitt (22) in den zweiten Leitungsabschnitt (24) und/oder aus dem zweiten Leitungsabschnitt (24) in den ersten Leitungsabschnitt (22) gelangen.

2. Vorrichtung (20) nach Anspruch 1,
wobei der dritte Leitungsabschnitt (26) einen zum ersten Leitungsabschnitt (22) hin abfallenden ersten Teilabschnitt (27) und/oder einen zum zweiten Leitungsabschnitt (24) hin abfallenden zweiten Teilabschnitt (28) umfasst, sodass Kondensat (K) aufgrund der Schwerkraft in mindestens einer Richtung aus dem dritten Leitungsabschnitt (26) abfließen kann.

3. Vorrichtung (20) nach Anspruch 2,
wobei der erste Teilabschnitt (27) und der zweite Teilabschnitt (28) in einem Übergangsabschnitt (29) ineinander übergehen;
wobei der erste Teilabschnitt (27) und der zweite Teilabschnitt (28) ausgehend vom Übergangsabschnitt (29) zum jeweiligen Leitungsabschnitt (22, 24) hin abfallen und/oder der Übergangsabschnitt (29) im betriebsfähigen Zustand der Vorrichtung (20) den höchsten Punkt des dritten Leitungsabschnitts (26) oder der Leitung (21) bildet.

4. Vorrichtung (20) nach Anspruch 2 oder 3,
wobei mindestens einer der beiden Teilabschnitte (27, 28) einen schrägen Abschnitt (30), dessen Längsrichtung im betriebsfähigen Zustand der Vorrichtung (20) schräg zur Horizontalen verläuft, und/oder einen vertikalen Abschnitt (31), dessen Längsrichtung im betriebsfähigen Zustand der Vorrichtung (20) vertikal verläuft, umfasst.

5. Vorrichtung (20) nach Anspruch 4,
wobei der vertikale Abschnitt (31) an einem seiner Enden mit dem jeweiligen Leitungsabschnitt (22, 24) und an seinem anderen Ende mit dem schrägen Abschnitt (30) verbunden ist.

6. Vorrichtung (20) nach einem der vorhergehenden Ansprüche, ferner umfassend:
eine Heizeinrichtung (40), die ausgebildet ist, um den dritten Leitungsabschnitt (26) auf eine erste Temperatur zum Vermeiden von Kondensation im dritten Leitungsabschnitt (26) und/oder auf eine zweite Temperatur zum Abtöten und/oder Inaktivieren von Keimen zu erwärmen.

7. Vorrichtung (20) nach einem der vorhergehenden Ansprüche, ferner umfassend:
eine Bestrahlungseinrichtung (42), die ausgebildet ist, um den dritten Leitungsabschnitt (26) mit einer Strahlung (43) zum Abtöten und/oder Inaktivieren von Keimen, insbesondere mit ultravioletter Strahlung, zu bestrahlen.

8. Vorrichtung (20) nach einem der vorhergehenden Ansprüche, ferner umfassend:
eine Auffangeinrichtung (44), die ausgebildet ist, um Kondensat (K) aus dem ersten Leitungsabschnitt (22) und/oder dem zweiten Leitungsabschnitt (24) aufzufangen und/oder abzuleiten, bevor es in den dritten Leitungsabschnitt (26) gelangt.

9. Vorrichtung (20) nach einem der vorhergehenden Ansprüche,
wobei die Leitung (21) zumindest im dritten Leitungsabschnitt (26) zumindest teilweise aus einem Metall und/oder einem Polymermaterial, insbesondere aus Polyamid, gefertigt ist und/oder zumindest im dritten Leitungsabschnitt (26) zumindest teilweise eine antiadhäsive und/oder antimikrobielle Innenfläche aufweist.

10. Vorrichtung (20) nach Anspruch 9,
wobei die Innenfläche durch eine Schicht gebildet ist, die mindestens eines der folgenden Materialien umfasst: Silber, Kupfer, Zink, Platin oder deren Legierungen.

11. Vorrichtung (20) nach einem der vorhergehenden Ansprüche, ferner umfassend:
ein Ventil (46), das ausgebildet ist, um einen Atemgasstrom durch die Leitung (21) vom ersten Anschluss (23) zum zweiten Anschluss (25) zu ermöglichen und vom zweiten Anschluss (25) zum ersten Anschluss (23) zu sperren.

12. Vorrichtung (20) nach einem der vorhergehenden Ansprüche, ferner umfassend:
einen Filter (48), der ausgebildet ist, um Keime aus dem durch die Leitung (21) strömenden Atemgas zu filtern.

13. Vorrichtung (20) nach Anspruch 12,
wobei der Filter (48) durch einen Schwebstofffilter und/oder einen chemischen Filter gebildet ist.

14. Vorrichtung (20) nach einem der vorhergehenden Ansprüche,
wobei die Leitung (21) eine Gesamtlänge zwischen 2 cm und 100 cm, bevorzugt zwischen 3 cm und 70 cm, besonders bevorzugt zwischen 3 cm und 51 cm, weiter bevorzugt zwischen 3 cm und 11 cm hat; und/oder
wobei der dritte Leitungsabschnitt (26) eine Länge zwischen 2 cm und 50 cm, bevorzugt zwischen 2 cm und 20 cm, besonders bevorzugt zwischen 2 cm und 5 cm, hat; und/oder
wobei der dritte Leitungsabschnitt (26) einen Durchmesser zwischen 5 mm und 50 mm, bevorzugt zwischen 8 mm und 40 mm, besonders bevorzugt zwischen 10 mm und 30 mm hat.

15. Beatmungssystem (100), umfassend:
eine Vorrichtung (20) nach einem der vorhergehenden Ansprüche;
wobei das Beatmungssystem (100) ferner umfasst:
ein Beatmungsgerät (1), wobei die Vorrichtung (20) über den ersten Anschluss (23) an das Beatmungsgerät (1) angeschlossen ist; und/oder
ein Atemgasleitungssystem (50) zum Leiten von Atemgas hin zu und/oder weg von einem Beatmungsgerät (1), wobei die Vorrichtung (20) über den zweiten Anschluss (25) an das Atemgasleitungssystem (50) angeschlossen ist.
